# EUROPÄISCHE PATENTSCHRIFT

(11) **EP 0 581 233 B1**
(45) Veröffentlichungstag und Bekanntmachung des Hinweises auf die Patenterteilung: **22.10.1997**
(21) Anmeldenummer: 93111949.9
(22) Anmeldetag: 26.07.1993
(51) Int. Cl.: A61F 2/24

(54) **Heterologe Mitral- oder Tricuspidal-Bioherzklappenprothese**
Heterologous mitral - or - tricuspidate cardiac valve bioprosthesis
Bioprothèse hétéroloque de valve cardiaque mitrale ou tricuspide

(30) Priorität: 28.07.1992 BR 9202905
(43) Veröffentlichungstag der Anmeldung: 02.02.1994
(73) Patentinhaber: BIOCOR INDUSTRIA E PESQUISA LTDA., Nova Lima 34000-000, MG (BR)
(72) Erfinder: Peredo, Mario Osvaldo Vrandecic, Belo Horizonte Minas Gerais (BR)
(74) Vertreter: Arendt, Helmut, Dipl.-Ing.

(56) Entgegenhaltungen:
- DE-A- 2 742 681
- DE-A- 3 442 088
- GB-A- 2 149 062

## Beschreibung

Die Erfindung bezieht sich auf Herzklappensubstitute, insbesondere auf solche, die in Mitral- und Tricuspidalposition benutzt werden, bestehend aus einem vollständigen Mitralklappenkomposit, das aus einem heterologen, biologischen Material besteht, und eine bessere Dauerhaltbarkeit sowie bessere hämodynamische Leistung in der Mitral- oder Tricuspidalposition aufweist, entsprechend den besonderen Erfordernissen der Strömung und der Kontraktilität des linken Ventrikels.

Rheumathische Herzerkrankungen treten immer noch endemisch in den Entwicklungsländern auf. Sie sind für Erkrankungen verantwortlich, die mit Herzklappen, insbesondere mit Mitralklappen, im Zusammenhang stehen, wovon im allgemeinen die jüngere Bevölkerung betroffen ist.

Funktionelle Folgen dieser Schädigungen sind Klappenstenosen, -insuffizienzen oder gemischte Schädigungen und explorative chirugische Eingriffe. Weiterhin kann, wegen der Schwere der Läsionen der Herzklappenbestandteile, eine wiederherstellende Klappenoperation ausgeschlossen sein. In diesem Fall kann ein Austausch oder eine Klappenersatzoperation mit Einsatz einer mechanischen Klappe oder einer Bioprothese (zusammengesetzt aus prothetischen und biologischen Materialien) erforderlich sein.

Mechanische Prothesen haben den Vorteil einer besseren Dauerhaltbarkeit und den Nachteil einer lebenslang erforderlichen Behandlung mit Anticoagulantien; dies in sich ist eine iatrogene Erkrankung neben anderen Komplikationen wie Hämorrhagien, Embolien und Thromboembolien.

Bioprothesen werden aus biologischem Gewebe hergestellt, welches auf einem vorher mit einem synthetischen Material beschichteten Gerüst befestigt wird, so daß ein in eine Richtung öffnendes Ventil entsteht, das in einem bestimmten Ausmaß die Leistung einer gesunden menschlichen Herzklappe reproduziert. Bioprothesen haben mehrere Vorteile, wie zentralen Durchfluß, zufriedenstellende Hämodynamik, bessere Lebensqualität, geringeres Auftreten von Thromboembolien, und Bioprothesen erfordern nicht den Einsatz von Anticoagulantien.

Der Hauptnachteil der Bioprothese ist ihre mangelnde Dauerhalbarkeit, hauptsächlich bei der jüngeren Bevölkerung, wegen des Verschleißes der biologischen Komponenten, welche hohen intraventriculären Drücken ausgesetzt ist, und, im Fall der Mitralklappe, ist die gegenwärtig gebräuchliche Ausführung nicht geeignet, den hohen Drücken und dem besonderen Strömungsbild "VORTEX" (Wirbel) des linken Ventrikels zu widerstehen.

Der natürliche Mitralklappenkomplex besteht aus Mitralring, Mitralklappensegel, Subvalvularapparat und Papillarmuskeln. Die harmonische Zusammenarbeit dieses Komplexes bestimmt die Angemessenheit seiner beabsichtigten Funktion.

Die gegenwärtig gebräuchlichen Mitralklappensubstitute (siehe z.B. GB-A-2 149 062) sind mit einem Klappensegel oder einer Scheibe an einem festen Rahmen oder unflexiblen Ring, ohne die subvalvulären Bestandteile und die Papillarmuskeln fixiert, welche als essentiell für die angestrebte Leistung des linken Ventrikels als Einheit angesehen werden.

Die gegenwärtig gebräuchlichen Mitralherzklappensubstitute arbeiten gut als in eine Richtung öffnende Ventile und erlauben adäquate Strömungscharakteristiken. Jedoch trägt das Fehlen des subvalvulären Apparates und der Papillarmuskeln, die fundamentale Elemente zur Erhaltung der linken Ventrikelfunktion sind, mit der Zeit zu negativen Folgeerscheinungen bei, z. B. Funktionsstörungen und Dilatationen des linken Ventrikels.

Es ist Aufgabe der Erfindung, ein dauerhafteres, heterologes, vollständig rahmenloses Mitralklappensubstitut zu schaffen, das für eine zufriedenstellende hämodynamische Leistung in der Mitral- oder Tricuspidalposition sorgt. Eine weitere Aufgabe der Erfindung ist es, ein Mitralklappensubstitut zu liefern, welches die Erfordernisse des linken Ventrikels, spezifische Strömung und Kontraktilität, erfüllt. Dieses Ziel wird durch ein heterologes, von Säugetieren, die in Größe, Anatomie und Funktion dem Menschen ähnlich sind, gewonnenes, vollständig gerüstfreies Mitralherzklappensubstitut zum Ersatz von Mitral- und Tricuspidalklappen, bestehend aus einem Mitralnahtring, Mitralklappensegeln und einem subvalvulären Apparat mit Sehnenfädenbefestigungen, die an dem heterologen Pericard oder synthetischen Material fixiert werden können, wobei die Sehnenfädenbefestigungen am Pericard oder dem synthetischen Material angeordnet werden können und die Sehnenfädenbefestigungen ferner zwischen zwei pericardialen Lappen, abhängig von der räumlichen Geometrie einer jeden Sehnenfädenbefestigung eines jeden Papillarmuskels, verankert werden können, erreicht. Die anatomische und physiologische Formgebung dieses heterologischen Komplexes bietet hämodynamische Verhältnisse für die spezifischen Erfordernisse der Strömung und Kontraktilität des linken Ventrikels beim Menschen. So vermeidet die Erfindung Komplikationen, wie Funktionsstörungen und Dilatationen des linken Ventrikels, dieses sorgt wiederum für eine bessere Lebensqualität des Empfängers und eine längere Dauerhaltbarkeit der Klappen.

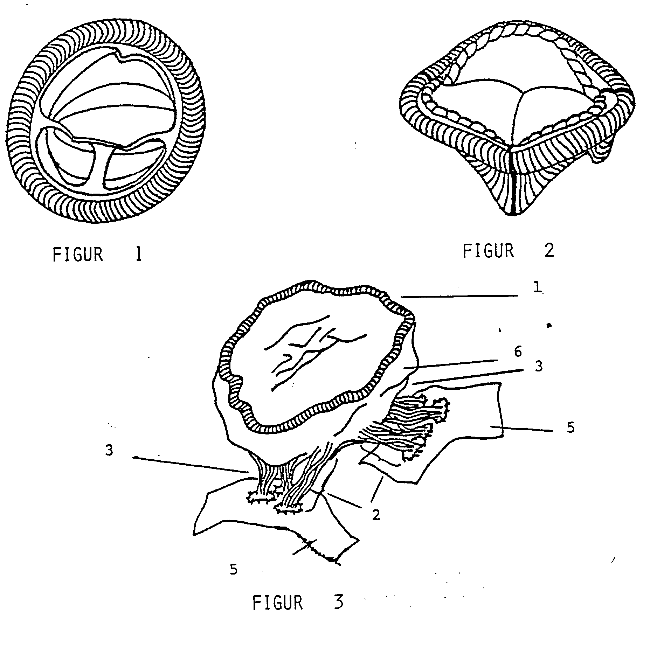

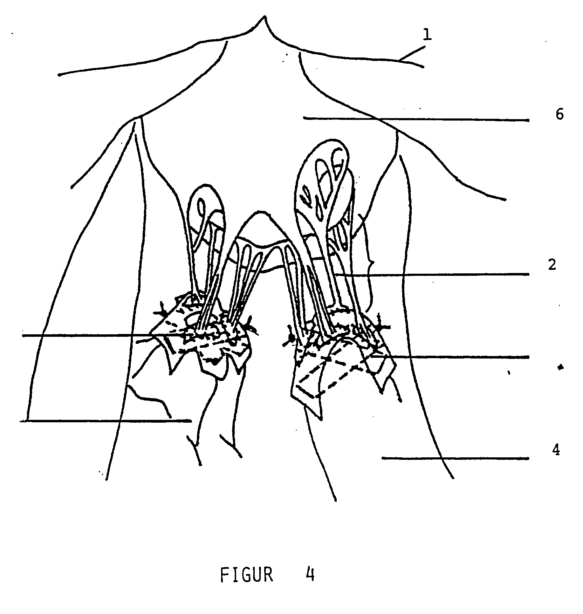

Die Erfindung wird nachstehend mit Bezug auf die beigefügten Zeichnungen beschrieben, in welchen Figur 1 eine mechanische Prothese nach dem Stand der Technik darstellt. Figur 2 zeigt eine Bioprothese, ebenfalls nach dem Stand der Technik. Figur 3 stellt eine Ausführungsform des heterologen, vollständig gerüstfreien Mitralklappensubstitutes der vorliegenden Erfindung zum Gebrauch in der Mitral- und Tricuspidalposition dar, und Figur 4 stellt das Mitralklappensubstitut der Figur 3 im Einsatz dar.

Wie in den Figuren 3 und 4 gezeigt, wird das heterologe, vollständig gerüstfreie Mitralklappensubstitut der vorhandenen Erfindung zur Benutzung in der Mitral- und Tricuspidalstellung dargestellt. Dieses Mitralklappensubstitut besteht aus einem Mitralnahtring 1, einem vollständigen subvalvulären Apparat 2 mit Sehnenbefestigungen 3, die an einem heterologen, pericardialen Lappentransplantat oder an einem synthetischen Material, abhängig von seiner Geometrie fixiert werden.

Dieser heterologe Komplex enthält die native Mitralklappe, die von jedem Säugetier, nach Größe, Anatomie und ähnlicher Funktionen vergleichbar mit dem menschlichen Herz, erhältlich ist, einschließlich z. B. vom Schwein, Schaf, Rind und anderen.

Dieser heterologe Komplex wird sofort nach dem Schlachten des Tieres durch chirugische Technik entfernt. Der gesamte Mitralkomplex wird gewonnen und in die balancierte, Elektrolyte und Glutaraldehyde enthaltende Lösung eingetaucht. Danach wird der überschuß an Muskelgewebe durch ein Sektionsverfahren entfernt. Jedes Verfahren wird so ausgeführt, daß die Unversehrtheit des Mitralkomplexes erhalten bleibt. Fortgesetzte Beobachtung der Qualitätskontrolle jeden Bestandteils wird täglich eingehalten, bis die geeignete "Gerbung" und Vernetzung des Gewebes durch die balancierte, Elektolyte und Glutaraldehyde enthaltende Lösung erreicht wird, wie es in dem brasilianischen Patent BR-A-8402134 (veröffentlicht am 14.8.84) beschrieben wurde, das demselben Anmelder der vorliegenden Erfindung erteilt wurde.

In diesem Komplex wird jede einzelne der Chordae und Sehnengruppen 3 erhalten und zwischen zwei pericardialen oder synthetischen Materialien unter Verwendung von Mono- oder Polyfilamentnahtmaterial fixiert. Dieses Verfahren verankert die Sehnengruppen an dieser neuen Insertion 3 in einer Sandwichart zwischen zwei pericardialen Lappen 5, wobei die räumliche Geometrie eines jeden Punktes der Insertion der einzelnen Chordae oder Chordaegruppen 3 jeden Papillarmuskels berücksichtigt wird.

Dieser vollständig biologische Komplex besteht aus einem Nahtring 1, Klappensegel 6 und dem subvalvulären Apparat 2, der Sehnenbefestigungen 3 hat, die an dem Pericard 5 oder einem synthetischen Material befestigt sind und als Herzklappensubstitut für die Mitral- und Tricuspidalklappen beim Menschen benutzt wird. Dieser Komplex präsentiert eine besondere Formgebung, die seine Verankerung an den Papillarmuskeln 4 und an dem Mitralring des Patienten ermöglicht und so für eine bessere hämodynamische Leistung sorgt und Funktionsstörungen und Dilatationen des linken Ventrikels verhindert. Als Ergebnis davon verbessert dieser Komplex die Lebensqualität des Empfängers als auch die Dauerhaltbarkeit des Komplexes. Dieses heterologe, vollständig gerüstfreie Mitralherzklappensubstitut kann eine bessere Dauerhaltbarkeit bieten, da es alle bekannten Bestandteile, die für eine harmonische Funktion der Mitralklappe und des linken Ventrikels notwendig sind, besitzt, welche in vorbekannten Herzklappensubstituten nicht vorhanden waren.

## Patentansprüche

1. Heterologes, von Säugetieren, die in Größe, Anatomie und Funktion dem Menschen ähnlich sind, gewonnenes, vollständig gerüstfreies Mitralherzklappensubstitut zum Ersatz von Mitral- und und Tricuspidalklappen, bestehend aus einem Mitralnahtring (1), Mitralklappensegeln (6) und einem subvalvulären Apparat (2) mit Sehnenfädenbefestigungen (3), die an dem heterologen Pericard (5) oder synthetischen Material fixiert werden können, wobei die Sehnenfädenbefestigungen (3) am Pericard (5) oder dem synthetischen Material angeordnet werden können und die Sehnenfädenbefestigungen (3) ferner zwischen zwei pericardialen Lappen, abhängig von der räumlichen Geometrie einer jeden Sehnenfädenbefestigung eines jeden Papillarmuskels (4), verankert werden können.

2. Herzklappensubstitut nach Anspruch 1, dadurch gekennzeichnet, daß die Säugetiere zu einer aus Schwein, Schaf und Rind bestehenden Gruppe gehören.

3. Herzklappensubstitut nach Anspruch 1, dadurch gekennzeichnet, daß jede genannte Sehnenfädenbefestigung (3) auf oder zwischen den pericardialen Lappen zur Sicherung und Fixierung mono- oder polyfilamenteuses Nahtmaterial aufweist.

## Claims

1. Heterologous mitral valve replacement which is obtained from mammals similar in size, anatomy and function to humans, is completely free from skeleton and is used for the replacement of mitral and tricuspid valves, consisting of a mitral suture ring (1), mitral valve leaflets (6) and a subvalvular apparatus (2) with tendinous cord attachments (3) which can be fixed on the heterologous pericardium (5) or synthetic material, where the tendinous cord attachments (3) can be arranged on the pericardium (5) or the synthetic material, and the tendinous cord attachments (3) can furthermore be anchored between two pericardial lobes, depending on the spatial geometry of each tendinous cord attachment of each papillary muscle (4).

2. Heart valve replacement according to Claim 1, characterized in that the mammals belong to a group comprising pigs, sheep and cattle.

3. Heart valve replacement according to Claim 1, characterized in that each said tendinous cord attachment (3) has monofilament or multifilament suture material on or between the pericardial lobes for securing and fixing.

## Revendications

1. Prothèse de valvule cardiaque mitrale hétérologue, obtenue de mammifères qui sont similaires aux humains en taille, anatomie et fonction, complètement exempte de soutien, pour remplacer les valvules mitrale et tricuspide, consistant en un anneau mitral de suture (1), des valves de valvule mitrale (6) et un appareil sous-valvulaire (2) avec des cordages fibreux (3) qui peuvent être fixés au péricarde hétérologue (5) ou à un matériau synthétique, où les cordages fibreux (3) peuvent être disposés sur le péricarde (5) ou le matériau synthétique et les cordages fibreux (3) peuvent, en outre, être ancrés entre deux lobes péricardiques, en fonction de la géométrie spatiale de chacun des cordages fibreux de chaque pilier du coeur (4).

2. Prothèse de valvule cardiaque suivant la revendication 1, caractérisée en ce que le mammifère appartient au groupe constitué des porcs, des moutons et des boeufs.

3. Prothèse de valvule cardiaque suivant la revendication 1, caractérisée en ce que chaque cordage fibreux (3) cité, présente sur ou entre les lobes péricardiques, un matériau mono- ou polyfilamenteux de suture pour l'implantation et la fixation.
